# EUROPEAN PATENT APPLICATION

(11) **EP 4 749 557 A1**
(43) Date of publication of application: **27.05.2026**
(21) Application number: 25188331.0
(22) Date of filing: 09.07.2025
(51) Int. Cl.: G06Q 30/018, G06Q 50/00, G06Q 50/26, G06V 10/82

(54) **FOOD WASTE COMPUTATION AND DISTRIBUTED PERSISTENCE**

(30) Priority: 25.11.2024 GR 20240100842
(71) Applicant: Konnecta Systems IKE, 14561 Kifisia (GR)
(72) Inventor: Mygiakis, Antonios, 15238 Chalandri (GR); Zafeiropoulou, Aristea Maria, 15127 Attica (GR); Niavis, Charilaos, 14122 Athens (GR); El Saer, Andreas, 16233 Athens (GR); Savva, Kalliopi, 35800 Volos (GR)
(74) Representative: Bringer IP

(57) **Abstract**

The quantification and persistence of imaged food includes the acquisition of imagery by an optical sensor of a digital camera such as that included within a smart phone. The optically sensed imagery is then loaded into memory and a food portion can be identified therewithin. Subsequently, a food waste value is computed of the identified food portion and the food waste value is encapsulated within a digital artifact to be written into the memory. Finally, the digital artifact is uploaded into a secure ledger entry from over a computer communications network, such as an entry in a blockchain ledger.

## Description

### BACKGROUND OF THE INVENTION

### Field of the Invention

The present invention relates to the technical field of image processing and recognition and more particularly to image processing food.

### Description of the Related Art

Food waste is food that is intended for human consumption that is wasted and lost. Food waste refers not only to food that consumers do not finish at the restaurant or at home, but also to the raw materials that may be lost during farming, harvesting processing, transportation, or storage. Thus, food waste can occur anywhere throughout the entire supply chain. Of note, nearly one-third of global food supplies are wasted or lost. Food waste has a significant impact upon the environment, the global economy and national economies, food security and nutrition. The increasing and persistent global demand for food is also driving fertile land useless while contributing to more land degradation and deforestation, which as a result, destroys our precious natural habitats and biodiversity, limiting the services that they provide and disrupting entire ecosystems.

Essential to the reduction in food waste, then, is the changing of consumer attitudes in the acquisition, consumption and disposition of prepared food. Indeed, the culture of food consumption seldom accounts for the likelihood that a complete, prepared meal will not be fully consumed leaving "left overs". As such, it is imperative that individual consumers consume prepared food with an awareness of and a goal to minimize the amount of prepared food remaining on the plate. Yet, the disconnected, isolated recognition of food waste on a consumer by consumer basis does not lend itself to a standardized approach to food waste reduction and behavior modification amongst food consumers.

### BRIEF SUMMARY OF THE INVENTION

Embodiments of the present invention address technical deficiencies of the art in respect to the image processing of food waste. To that end, embodiments of the present invention provide for a novel and non-obvious method for the quantification and persistence of imaged food waste. Embodiments of the present invention also provide for a novel and non-obvious computing device adapted to perform the foregoing method. Finally, embodiments of the present invention provide for a novel and non-obvious data processing system incorporating the foregoing device in order to perform the foregoing method.

In one embodiment of the invention, a method for quantifying and persisting imaged food includes the acquisition of imagery by an optical sensor of a digital camera such as that included within a smart phone. The optically sensed imagery is then loaded into memory and a food portion can be identified therewithin. Subsequently, a food waste value is computed of the identified food portion and the food waste value is encapsulated within a digital artifact to be written into the memory. Finally, the digital artifact is uploaded into a secure ledger entry from over a computer communications network, such as an entry in a blockchain ledger.

In one aspect of the embodiment, the identifying of the food portion is a segmentation of optically sensed imagery into different food portions and a selection of one of the different food portions.

In another aspect of the embodiment, the food waste value is a determination of remaining mass and volume of the food portion relative to an extrapolated mass and volume of an estimation of an initial food portion prior to consumption.

In yet another aspect of the embodiment, the determination of the remaining mass and volume of the food portion is a submission of the food portion to a convolutional neural network trained to predict mass and volume values for imagery of food portions present in imagery.

Other aspects of the embodiment include:
- A transformation of the digital artifact into an immutable icon and a transmission of the immutable icon to the smart phone over the computer communications network.
- The uploading of the food waste value to a social media posting of an end user associated with the smart phone.

In another embodiment of the invention, a data processing system is adapted for quantifying and persisting imaged food waste. The system includes a host computing platform of one or more computers, each with memory and one or processing units including one or more processing cores. The system also includes a digital camera coupled to the host computing platform. Finally, the system includes a food waste quantification and persistence module. The module includes computer program instructions enabled while executing in the memory of at least one of the processing units of the host computing platform to load imagery into memory that has been optically sensed by the digital camera, to identify a food portion within the optically sensed imagery, to compute a food waste value of the identified food portion, to write a digital artifact into the memory encapsulating the computed food waste value and to upload the digital artifact into a secure ledger entry from over a computer communications network.

In even yet another embodiment, a computing device includes a non-transitory computer readable storage medium having program instructions stored therein. The instructions are executable by at least one processing core of a processing unit in order to cause the processing unit to quantify and persist imaged food waste. Specifically, the program instructions load optically sensed imagery into memory, identify a food portion within the optically sensed imagery, compute a food waste value of the identified food portion, write a digital artifact into the memory encapsulating the computed food waste value and upload the digital artifact into a secure ledger entry from over a computer communications network.

In this way, the technical deficiencies of the desire to reduce food waste through changes in consumer behavior are overcome owing to the image capture of a completed meal and the automated computation of a food waste value strictly from an analysis of the imagery in order to facilitate the application of rules to generate optimized food shopping lists, propose dietary changes by the consumer, to publicize the state of food waste optimization to one's peers, or even to mint a visual certification of food waste minimization excellence..

Additional aspects of the invention will be set forth in part in the description which follows, and in part will be obvious from the description, or may be learned by practice of the invention. The aspects of the invention will be realized and attained by means of the elements and combinations particularly pointed out in the appended claims. It is to be understood that both the foregoing general description and the following detailed description are exemplary and explanatory only and are not restrictive of the invention, as claimed.

### BRIEF DESCRIPTION OF THE SEVERAL VIEWS OF THE DRAWINGS

The accompanying drawings, which are incorporated in and constitute part of this specification, illustrate embodiments of the invention and together with the description, serve to explain the principles of the invention. The embodiments illustrated herein are presently preferred, it being understood, however, that the invention is not limited to the precise arrangements and instrumentalities shown, wherein:
Figure 1 is a pictorial illustration reflecting different aspects of a process of quantifying and persisting imaged food waste;
Figure 2 is a block diagram depicting a data processing system adapted to perform one of the aspects of the process of Figure 1; and,
Figure 3 is a flow chart illustrating one of the aspects of the process of Figure 1.

### DETAILED DESCRIPTION OF THE INVENTION

Embodiments of the invention provide for quantifying and persisting imaged food waste. In accordance with an embodiment of the invention, imagery of consumed food is captured in association with an end user and the imagery is segmented into different portions of the consumed food. One or more of the segments is then classified according to weight, volume or both and a food waste metric is computed based upon the classification of the segments. The food waste metric is then incorporated into a digital artifact and uploaded to a secure ledger in connection with the end user. Consequently, a food waste reduction incentivization process accessing the digital artifact can produce a reduction food waste, for instance, based upon a rules driven recommendation for food shopping or diet based upon the food waste metric, a posting to social media for the end user with the food waste metric, or through a certification of food waste optimization embodied within a non-fungible token formulated with the food waste metric for the end user.

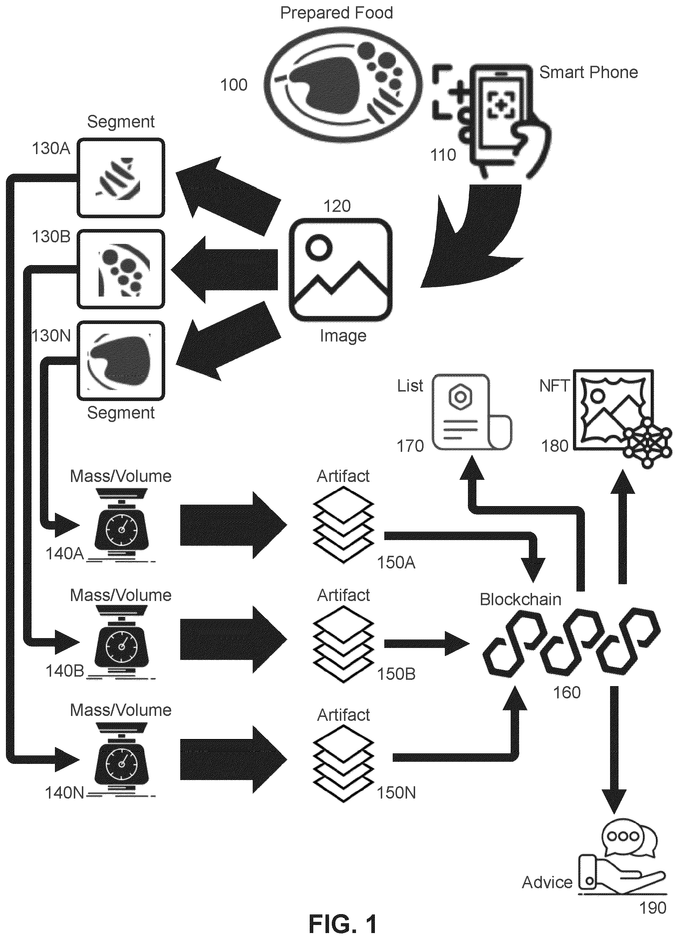

In illustration of one aspect of the embodiment, Figure 1 pictorially shows a process of quantifying and persisting imaged food waste. As shown in Figure 1, a digital camera on a smart phone 110 captures imagery 120 of a consumed plate of prepared food 100. The imagery 120 is segmented by portion into different segments 130A, 130B, 130N. Then, a corresponding mass/volume metric 140A, 140B, 140N is determined for each of the segments 130A, 130B, 130N and transformed into a food waste value that is incorporated into respectively different digital artifacts 150A, 150B, 150N. Then, the digital artifacts 150A, 150B, 150N are uploaded to a remotely disposed secure ledger 160, such as the blockchain, so that the artifacts 150A, 150B, 150N can be accessed by third party processes in order to generate a list 170 such as a shopping list or diet instructions based upon an application of one or more rules to the food waste value, to mint an associated non-fungible token (NFT) 180 certifying an optimization in food waste reduction based upon the food waste value, or advice 190 generated based upon the application of one or more rules to the food waste values of the digital artifacts 150A, 150B, 150N.

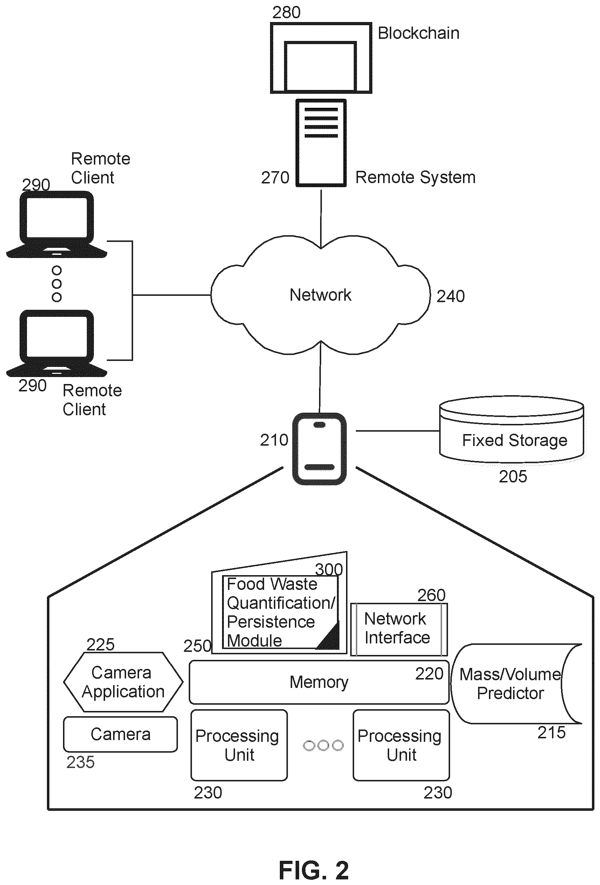

Aspects of the process described in connection with Figure 1 can be implemented within a data processing system. In further illustration, Figure 2 schematically shows a data processing system adapted to perform the quantification and persistence of imaged food waste. In the data processing system illustrated in Figure 1, a host computing platform is provided. The host computing platform includes a smartphone 210, with memory 220 and one or more processing units 230. The smartphone 210 also includes fixed storage 205 into which data is persisted and is adapted for communication through network interface 260 with remote clients 290 over a data communications network 240.

The smartphone 210 also includes a camera 235 driven by camera application 225 and adapted to capture imagery upon receipt of a directive through a user interface to the camera application 225. Even further, the smartphone 210 includes a mass/volume predictor 215 configured to predict a mass/volume value responsive to the receipt of an image of a consumed food portion. In this regard, the mass/volume predictor 215 can be a convolutional neural network trained to associate specific imagery of specific food portions of known mass/volume values with the known mass/volume values.

Notably, a computing device 250 including a non-transitory computer readable storage medium can be included with the data processing system 200 and accessed by the processing units 230 of one or more of the computers 210. The computing device stores 250 thereon or retains therein a program module 300 that includes computer program instructions which when executed by one or more of the processing units 230, performs a programmatically executable process for quantifying and persisting imaged food waste. Specifically, the program instructions during execution processes captured imagery by the camera application 225 and segments the captured imagery by different food types in order to produce separate images for each consumed portion of one of the different food types of the captured imagery.

The program instructions then submit a selected one of the segments to the mass/volume predictor 215 and receive in response, a prediction of mass/volume for a consumed portion of food imaged within the selected one of the segments. Based upon the prediction of mass/volume, a food waste value is computed, for instance as a proportion of the mass/volume of the consumed portion relative to a known mass/volume of the portion of food prior to consumption. The program instructions then construct a digital artifact, e.g. a data structure disposed in a document encapsulating the food waste value and a corresponding identity of the end user consuming the consumed portion of food. Thereafter, the program instructions tokenize the digital artifact and upload the tokenized digital artifact over the data communications network 240 to blockchain 280 accessible through a remote system 270.

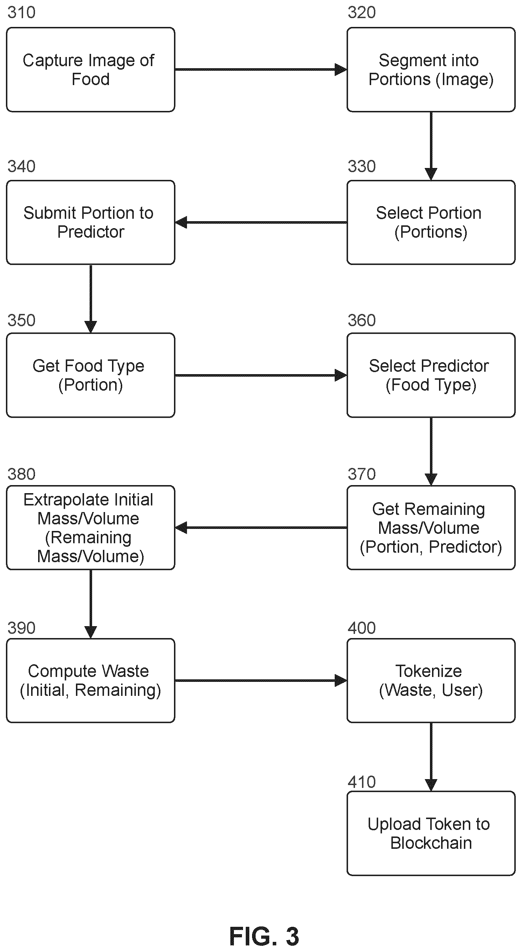

In further illustration of an exemplary operation of the module, Figure 3 is a flow chart illustrating one of the aspects of the process of Figure 1. Beginning in block 310, imagery is capture of a consumed plate of food and the imagery is then segmented in block 320 into different portions. Then, in block 330, one of the portions is selected for processing and in block 340, the selected portion is submitted to a predictor which predicts a mass/volume for a consumed food portion visually reflected within the one of the portions. In block 350, a food type for the consumed food portion is determined and in block 360, a predictor is selected which had been trained upon imagery of consumed portions of the determined food type.

In block 380, an initial mass/volume for the food portion in an unconsumed state is extrapolated from the imagery of the selected portion and in block 390, a waste value is computed for the consumed food portion, for instance as a ratio of the mass/volume of the consumed portion to a mass/volume known a priori of the food portion of the unconsumed state. The waste value is then stored in a digital artifact. Finally, in block 400, artifact is tokenized along with the identity of the end user and the tokenized artifact is uploaded to the blockchain in block 410.

Of import, the foregoing flowchart and block diagram referred to herein illustrate the architecture, functionality, and operation of possible implementations of systems, methods, and computing devices according to various embodiments of the present invention. In this regard, each block in the flowchart or block diagrams may represent a module, segment, or portion of instructions, which includes one or more executable instructions for implementing the specified logical function or functions. In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

More specifically, the present invention may be embodied as a programmatically executable process. As well, the present invention may be embodied within a computing device upon which programmatic instructions are stored and from which the programmatic instructions are enabled to be loaded into memory of a data processing system and executed therefrom in order to perform the foregoing programmatically executable process. Even further, the present invention may be embodied within a data processing system adapted to load the programmatic instructions from a computing device and to then execute the programmatic instructions in order to perform the foregoing programmatically executable process.

To that end, the computing device is a non-transitory computer readable storage medium or media retaining therein or storing thereon computer readable program instructions. These instructions, when executed from memory by one or more processing units of a data processing system, cause the processing units to perform different programmatic processes exemplary of different aspects of the programmatically executable process. In this regard, the processing units each include an instruction execution device such as a central processing unit or "CPU" of a computer. One or more computers may be included within the data processing system. Of note, while the CPU can be a single core CPU, it will be understood that multiple CPU cores can operate within the CPU and in either instance, the instructions are directly loaded from memory into one or more of the cores of one or more of the CPUs for execution.

Aside from the direct loading of the instructions from memory for execution by one or more cores of a CPU or multiple CPUs, the computer readable program instructions described herein alternatively can be retrieved from over a computer communications network into the memory of a computer of the data processing system for execution therein. As well, only a portion of the program instructions may be retrieved into the memory from over the computer communications network, while other portions may be loaded from persistent storage of the computer. Even further, only a portion of the program instructions may execute by one or more processing cores of one or more CPUs of one of the computers of the data processing system, while other portions may cooperatively execute within a different computer of the data processing system that is either co-located with the computer or positioned remotely from the computer over the computer communications network with results of the computing by both computers shared therebetween.

The corresponding structures, materials, acts, and equivalents of all means or step plus function elements in the claims below are intended to include any structure, material, or act for performing the function in combination with other claimed elements as specifically claimed. The description of the present invention has been presented for purposes of illustration and description but is not intended to be exhaustive or limited to the invention in the form disclosed. Many modifications and variations will be apparent to those of ordinary skill in the art without departing from the scope and spirit of the invention. The embodiment was chosen and described in order to best explain the principles of the invention and the practical application, and to enable others of ordinary skill in the art to understand the invention for various embodiments with various modifications as are suited to the particular use contemplated.

Having thus described the invention of the present application in detail and by reference to embodiments thereof, it will be apparent that modifications and variations are possible without departing from the scope of the invention defined in the appended claims as follows:

## Claims

1. A method for quantifying and persisting imaged food waste comprising:
loading optically sensed imagery into memory;
identifying a food portion within the optically sensed imagery;
computing a food waste value of the identified food portion;
writing a digital artifact into the memory encapsulating the computed food waste value; and,
uploading the digital artifact into a secure ledger entry from over a computer communications network.

2. The method of claim 1, wherein the optically sensed imagery is acquired from a digital camera embedded in a smart phone.

3. The method of claim 1, wherein the identifying of the food portion is a segmentation of optically sensed imagery into different food portions and a selection of one of the different food portions.

4. The method of claim 1, wherein the food waste value is a determination of remaining mass and volume of the food portion relative to an extrapolated mass and volume of an estimation of an initial food portion prior to consumption.

5. The method of claim 1, wherein the secure ledger entry is an entry in a blockchain ledger.

6. The method of claim 2, further comprising transforming the digital artifact into an immutable icon and transmitting the immutable icon to the smart phone over the computer communications network.

7. The method of claim 2, further comprising uploading the food waste value to a social media posting of an end user associated with the smart phone.

8. The method of claim 4, wherein the determination of the remaining mass and volume of the food portion is a submission of the food portion to a convolutional neural network trained to predict mass and volume values for imagery of food portions present in imagery.

9. A data processing system adapted for quantifying and persisting imaged food waste the system comprising:
a host computing platform comprising one or more computers, each with memory and one or processing units including one or more processing cores;
a digital camera coupled to the host computing platform; and,
a food waste quantification and persistence module comprising computer program instructions enabled while executing in the memory of at least one of the processing units of the host computing platform to perform:
load imagery into memory that has been optically sensed by the digital camera;
identify a food portion within the optically sensed imagery;
compute a food waste value of the identified food portion;
write a digital artifact into the memory encapsulating the computed food waste value; and,
upload the digital artifact into a secure ledger entry from over a computer communications network.

10. A computing device comprising a non-transitory computer readable storage medium having program instructions stored therein, the instructions being executable by at least one processing core of a processing unit to cause the processing unit to perform a method for quantifying and persisting imaged food waste, the method including:
loading optically sensed imagery into memory;
identifying a food portion within the optically sensed imagery;
computing a food waste value of the identified food portion;
writing a digital artifact into the memory encapsulating the computed food waste value; and,
uploading the digital artifact into a secure ledger entry from over a computer communications network.
